# EUROPÄISCHE PATENTSCHRIFT

(11) **EP 2 359 131 B1**
(45) Veröffentlichungstag und Bekanntmachung des Hinweises auf die Patenterteilung: **21.11.2012**
(21) Anmeldenummer: 09760842.6
(22) Anmeldetag: 24.11.2009
(51) Int. Cl.: G01N 27/62, G01N 33/00

(54) **VORRICHTUNG ZUR SAMMLUNG VON STARK ELEKTRONENAFFINEN PARTIKELN**
DEVICE FOR COLLECTING PARTICLES THAT HAVE A STRONG ELECTRON AFFINITY
DISPOSITIF POUR COLLECTER DES PARTICULES À FORTE AFFINITÉ ÉLECTRONIQUE

(30) Priorität: 26.11.2008 DE 102008059113
(43) Veröffentlichungstag der Anmeldung: 24.08.2011
(73) Patentinhaber: EADS Deutschland GmbH, 85521 Ottobrunn (DE)
(72) Erfinder: BEER, Sebastian, 81669 München (DE); MÜLLER, Gerhard, 85567 Grafing (DE); SPANNHAKE, Jan, D-86567 Hilgertshausen-Tandern (DE); LEGNER, Wolfgang, 85635 Höhenkirchen-Siegerstbrunn (DE)
(74) Vertreter: Rasch, Michael
(86) Internationale Anmeldenummer: PCT/EP2009/065740
(87) Internationale Veröffentlichungsnummer: WO 2010/060905

(56) Entgegenhaltungen:
- EP-A2- 1 059 521
- WO-A1-2008/111403

## Beschreibung

Die Erfindung betrifft eine Vorrichtung zur Sammlung von stark elektronenaffinen Partikeln, insbesondere von Sprengstoffpartikeln aus einem Gas. Gattungsgemäße Vorrichtungen, jedoch zur Sammlung von Partikeln aus Abgasen, werden in EP1059521 und WO2008/111403 offenbart.

Die zunehmende Verwendung von Sprengstoffen bei terroristischen Anschlägen ist ein allgemein erkanntes Sicherheitsproblem. Die Sicherung ziviler und militärischer Infrastrukturen gegen Personen und Fahrzeuge, die improvisierte Sprengkörper transportieren, stellt derzeit eine große Herausforderung dar. Hinweise auf verborgenen Sprengstoff können mit Hilfe sogenannter "BULK-Verfahren" (Röntgen-, Terrahertz-, NMR-Detektion) erhalten werden. Eine andere Klasse der Nachweisführung betrifft sogenannte "Trace-Detektion". Bei der Trace-Detektion werden Gase nachgewiesen, die von den verborgenen Sprengstoffen herrühren. Neben Spürhunden dienen hierzu vor allem Ionen-Mobilitäts-Spektrometer (IMS), die Spurengase im Konzentrationsbereich von ppb (10⁻⁹) nachweisen. Bei der IMS-Detektion von Sprengstoff wird insbesondere von der Tatsache Gebrauch gemacht, dass die meisten Sprengstoffe in ihrer Molekülstruktur stark elektronenaffine Nitro-Gruppen enthalten. Ein wichtiges Unterscheidungskriterium gegenüber anderen vorkommenden Spurenstoffen ist deshalb, dass Sprengstoffe sehr leicht negative Ionen bilden. Diese Stoffe können dann bei einer IMS-Messung noch über verschiedene Laufzeiten in der IMS-Driftstrecke unterschieden werden. Ein Problem bei einem derartigen Gasnachweis besteht darin, dass viele Sprengstoffe, darunter besonders die militärisch wichtigen Materialien, einen außerordentlich niedrigen Dampfdruck aufweisen, was ihre Nachweisbarkeit bei der Gasdetektion stark einschränkt.

Die Aufgabe der vorliegenden Erfindung ist es also, eine Vorrichtung zur Sammlung von stark elektronenaffinen Partikeln, insbesondere von Sprengstoffpartikeln, aus einem Gas bereit zu stellen, die auch eine Sammlung mikroskopisch kleiner Partikel mit einem Durchmesser im Bereich von 0,1 µ - 10 µ aus einer sehr viel größeren Menge analytisch uninteressanter Partikel von einem verdächtigen Objekt ermöglicht. Denn wegen der geringen Konzentration relevanter Partikel können die analytisch irrelevanten Begleitpartikel potentiell große Hintergrundsignale bei der IMS-Detektion erzeugen, die ein sicheres Erkennen dieser Partikel, insbesondere von Sprengstoffpartikeln, erschweren bzw. gänzlich unmöglich machen.

Erfindungsgemäß wird diese Aufgabe durch die in Anspruch 1 angegebenen Merkmale gelöst. Vorteilhafte Weiterbildungen des Erfindungsgedankens ergeben sich aus den Unteransprüchen und der Beschreibung.

Die erfindungsgemäße Vorrichtung ermöglicht die Trennung von Partikeln mit hoher Elektronenaffinität aus der umgebenden Luft und deren Sammlung, so dass diese dann in einem nachfolgenden Schritt, insbesondere durch Verdampfung, in nachweisbare Gase überführt werden können. Dabei wird der Gasstrom, insbesondere der Luftstrom, mit den Partikeln in ein elektrisches Feld geleitet, wobei an mindestens einer Ionisierungselektrode eine Koronaentladung erfolgt, bei der Elektronen bereit gestellt werden, welche die elektronenaffinen Moleküle der umgebenden Luft, insbesondere des Sauerstoffs, ionisieren und somit ein Plasma erzeugen. Partikel mit einer höheren Elektronenaffinität als diese Ionen entnehmen diesen die überschüssigen Elektronen und werden dadurch beladen. Die Beladung durch die Koronaentladung erfolgt also indirekt. Diese dann negativ geladenen Partikel werden vom elektrischen Feld zur positiv geladenen Kollektorelektrode hin beschleunigt werden. Dort werden die Partikel gesammelt.

Gemäß der Erfindung weist die Kollektorelektrode dazu eine elektrisch isolierende Schicht auf, auf der sich die Partikel ansammeln, die dann nach einer gewissen Zeit von der Schicht abgetragen oder mittels Erhitzer direkt auf der Schicht verdampft werden. Eine solche elektrisch isolierende Schicht kann ein separates Substrat, beispielsweise aus oxidiertem Silizium sein.

Die Kollektorelektrode ist in einer zweiten Ausführungsform in einem gasdurchströmten Querkanal angeordnet, in dem die auf die Kollektorelektrode auftreffenden Partikel dort neutralisiert und anschließend mittels des durch den Querstromkanal geleiteten Gases mitgenommen und einer nachfolgenden Analyseeinrichtung zugeführt werden.

Bei einer dritten Ausführungsform der Erfindung sind im Strömungskanal zwischen zwei außenseitigen Ionisierungselektroden zwei Elektroden-Längsgitter angeordnet, wobei zwischen und stromab dieser Elektroden-Längsgitter die Kollektorelektrode angeordnet ist. Durch aufeinander abgestimmte Spannungsverläufe an den vier Elektroden erfolgt dabei eine Ablenkung der elektrisch negativ beladenen Partikel zur Mitte, also in den Bereich zwischen den Elektroden-Längsgittern, von wo diese dann auf die Kollektorelektrode treffen.

Bei einer vierten Ausführungsform der Erfindung ist im Strömungskanal mindestens ein dazu konzentrischer, negativ geladener Ionisationsspitzenring vorhanden, dessen stromabwärtige Kante mit Ionisationsspitzen versehen ist, und weiter stromab mindestens ein negativ geladener Feldring, wobei ferner konzentrisch in der Mitte des Strömungskanals eine stabförmige, passivierte Kollektorelektrode angeordnet ist. Bei dieser Ausführungsform werden die elektrisch negativ beladenen Partikel nach der Aufladung nach radial innen bewegt und dort entweder auf der passivierten Kollektorelektrode angereichert oder in ein Abzugsrohr geleitet.

Bei einer fünften Ausführungsform der Erfindung ist die Wandung des rotationssymmetischen Strömungskanals als Kollektorelektrode ausgebildet und darin ist zentrisch die stabförmig ausgebildete Ionisierungselektrode angeordnet, wobei die Strömungskanalwandung in mindestens einem ringförmigen Abschnitt einen Radialauslass aufweist, in dem eine gasdurchlässige Auffangschicht vorgesehen ist, die für die Partikel weitgehend undurchlässig ist. Die angesammelten Partikel werden dann in einem Spülschritt aus der vorzugsweise als Vlies gebildeten Auffangschicht durch einen im Querstrom durchgeleitetes Spülgasstrom mitgerissen und anschließend einer Analyse zugeführt.

Die Erfindung wird nachfolgend anhand der beigefügten Zeichnungen weiter erläutert. Dabei zeigt:
- Figur 1:: eine erste Ausführungsform der Erfindung mit schematischer Darstellung;
- Figur 2:: die Ausführung gemäß Figur 1 in teilgeschnittener perspektivischer Darstellung;
- Figur 3:: eine zweite Ausführungsform der Erfindung in perspektivischer Darstellung (ohne Darstellung der Wandung);
- Figur 4:: eine schematische Darstellung der Elektrodenanordnung in der Ausführung gemäß Figur 3;
- Figur 5:: eine Darstellung der Spannungsverläufe in den Elektroden gemäß Figur 4;
- Figur 6:: eine Darstellung der Partikelwanderung zwischen der Elektrodenanordnung gemäß Figur 4 bei angelegter Spannung gemäß Figur 5;
- Figur 7:: eine schematische Schnittdarstellung einer dritten Ausführungsform der Erfindung;
- Figur 8:: ein Detail aus Figur 7;
- Figur 9:: eine schematische Prinzipdarstellung einer vierten Ausführungsform der Erfindung;
- Figur 10:: eine teilgeschnittene perspektivische Ansicht der Ausführung gemäß Figur 9.

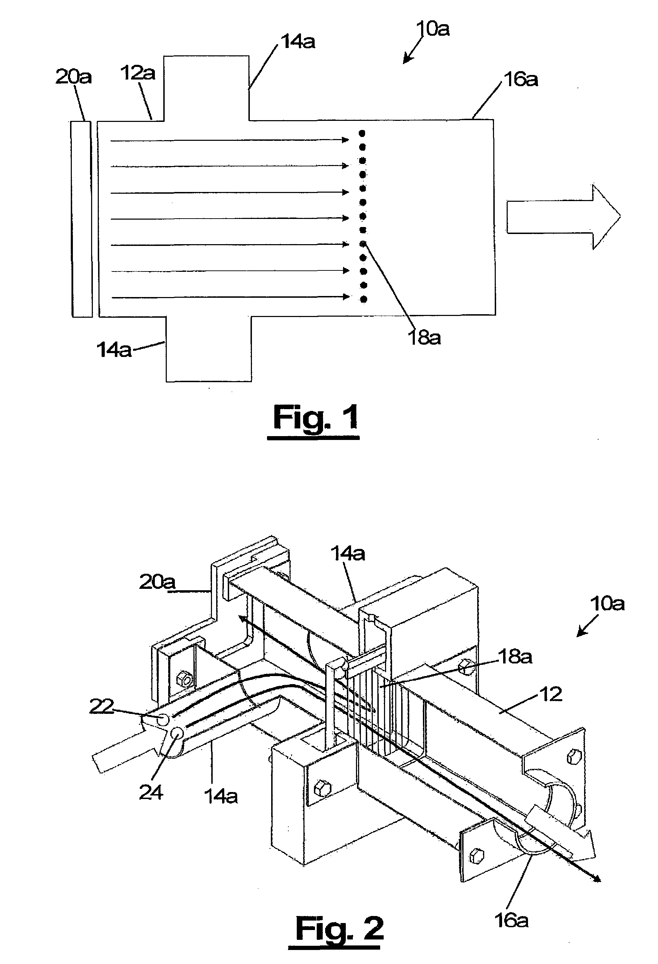

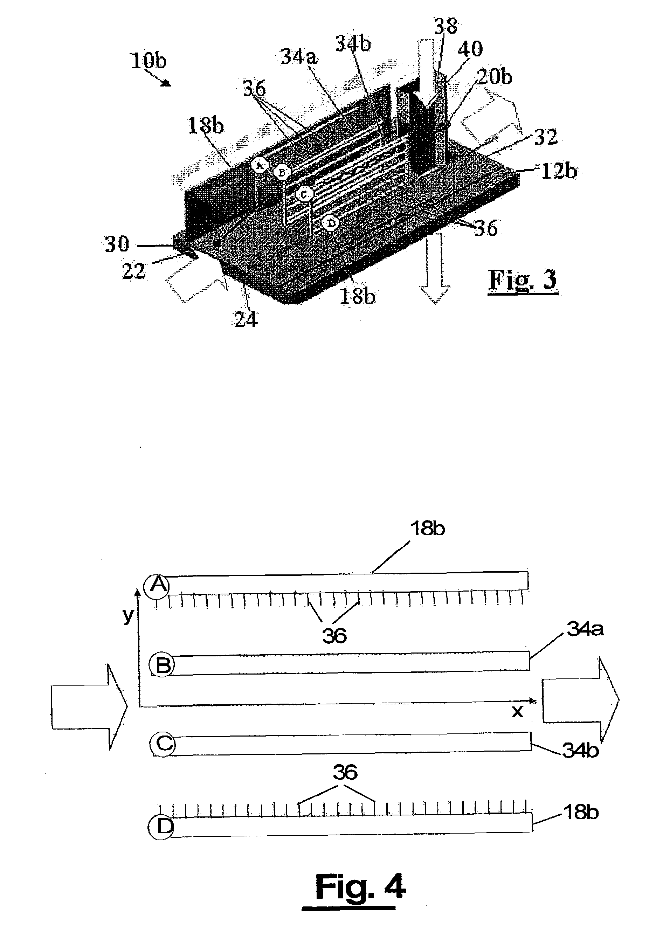

In den **Figuren 1 und 2** ist eine erste bevorzugte Ausführungsform einer Partikelsammelvorrichtung **10a** dargestellt und zwar in Figur 1 schematisch und in Figur 2 als teilgeschnittene perspektivische Darstellung. Diese Partikelsammelvorrichtung **10a** besteht im Wesentlichen aus einem Strömungskanal **12a** und zwei sich bezüglich dieses Strömungskanals **12a** gegenüberliegenden Gaseinlaufkanälen **14a.** Zwischen diesen Einlaufkanälen **14a** und dem stromabseitigen offenen Ende **16a** des Strömungskanals **12a** ist ein Drahtgitter als Ionisierungselektrode **18a** quer im Strömungskanal **12a** angeordnet. Am Austrittsende **16a** des Strömungskanals **12a** gegenüberliegenden Ende des Strömungskanals **12a** ist eine Kollektorelektrode **20a** vorgesehen, die den Strömungskanal **12a** in diese Richtung abschließt. Die Kollektorelektrode **20a** kann entweder abnehmbar sein oder diese kann mit einer Abstreifeinrichtung versehen sein, mittels der anhaftende Partikel entfernt werden können. Alternativ könnte die Kollektorelektrode **20a** auch eine Gestaltung aufweisen, wie diese in Figur 3 nachfolgend dargestellt und erläutert ist.

Im Betrieb wird mittels nicht dargestellter Ansaugeinrichtungen ein Luftstrom mit stark elektronenaffinen Partikeln in die Gaseinlaufkanäle **14a** eingesaugt und durch den Strömungskanal **12a** (in Figur 1 nach rechts) umgeleitet, umströmt das Drahtgitter **18a** und tritt am Strömungskanalauslass **16a** wieder aus.

Beide Partikel sind zunächst elektrisch neutral; eins ist stark elektronenaffin, das andere weist eine geringe Elektronenaffinität auf.

Zwischen der Ionisierungselektrode **18a** und der Kollektorelektrode **20a** ist ein Spannungspotential im Bereich von 5000 Volt, wobei die Ionisierungselektrode **18a** negativ und die Kollektorelektrode **20a** positiv aufgeladen ist. In Figur 2 ist ein elektronenaffiner Partikel **22** und ein neutraler Partikel **24** nebeneinander mit den Flugbahnen nach Eintritt in die Partikelsammelvorrichtung **10a** dargestellt. Dabei ist erkennbar, dass der neutrale Partikel **24** nach Passieren des Gaseinlaufkanals **14a** in den Strömungskanal **12a** eintritt und unbeeinflußt durch das Drahtgitter **18a** hindurch tritt um beim Strömungskanalauslass **16a** den Strömungskanal **12a** zu verlassen. Ein stark elektronenaffiner Partikel **22** hingegen bewegt sich nach Eintritt in den Strömungskanal **12a** in Richtung des Drahtgitters **18a** und erfährt aufgrund der hohen Feldstärke in der Umgebung der einzelnen negativ geladenen Geräte des Drahtgitters **18a,** welche eine Koroanaentladung erzeugt, eine indirekte Oberflächenentladung wobei diese Partikel sich mit Elektronen aufladen und daraufhin vom elektrischen Feld zwischen Ionisierungselektrode **18a** und Kollektorelektrode **20a** in Richtung der Kollektorelektrode **20a** beschleunigt werden. Die stark elektronenaffinen Partikel lagern sich auf der Oberfläche der Kollektorelektrode **20a** an. Dann kann diese Kollektorelektrode entnommen und in das thermische Desorptionssystem eines Ionen-Mobilitäts-Spektrometers (IMS) überführt und dort dann die chemische Zusammensetzung bestimmt werden.

Die Kollektorelektrode **20a** ist mit einer elektrisch isolierenden Schicht versehen um zu verhindern, dass diese sich an der positiv geladenen Elektrode wieder entladen und vielmehr sich dort anreichern. In den Figuren 3 und 4 ist eine zweite Ausführungsform der Erfindung dargestellt und zwar in Figur 3 in perspektivischer Ansicht ohne Darstellung des Strömungskanals und in **Figur 4** eine schematische Ansicht der vier Elektroden-Längsgitter.

Bei dieser Ausführungsform der Partikelsammelvorrichtung **10b** ist ein in Querschnitt rechteckiger Strömungskanal **12b** vorhanden, von dem in Figur 3 nur der Boden und eine Seitenwand dargestellt ist. Die zweite Seitenwand und die Deckwandung fehlen. Der Strömungskanal **12b** weist einen Einlass **30** und einen Auslass **32** auf, zwischen denen zwei Längsgitter **34a, 34b** beabstandet angeordnet sind. Entlang den beiden Seitenwänden sind zwei Ionisierungselektroden **18b** vorgesehen, die eine Anzahl von in den Strömungskanal **12b** hereinragenden Ionisierungsspitzen **36** aufweisen, an denen aufgrund der erhöhten Feldstärke Koronaentladungen erfolgen, welche die stark elektronenaffinen Partikel indirekt mit Ladungen beaufschlagen. Stromab zwischen den beiden Elektrodenlängsgittern **34a, 34b** ist ein Querstromkanal **38** mit einer Öffnung **40** angeordnet, in dessen Inneren die Kollektorelektrode **20b** angeordnet ist. Der Querstromkanal **38** ist von einem Gas (vorzugsweise Luft) durchströmt, die durch die Öffnung **40** eintretende Partikel mitreißt und einer Sammeleinrichtung zugeführt werden kann.

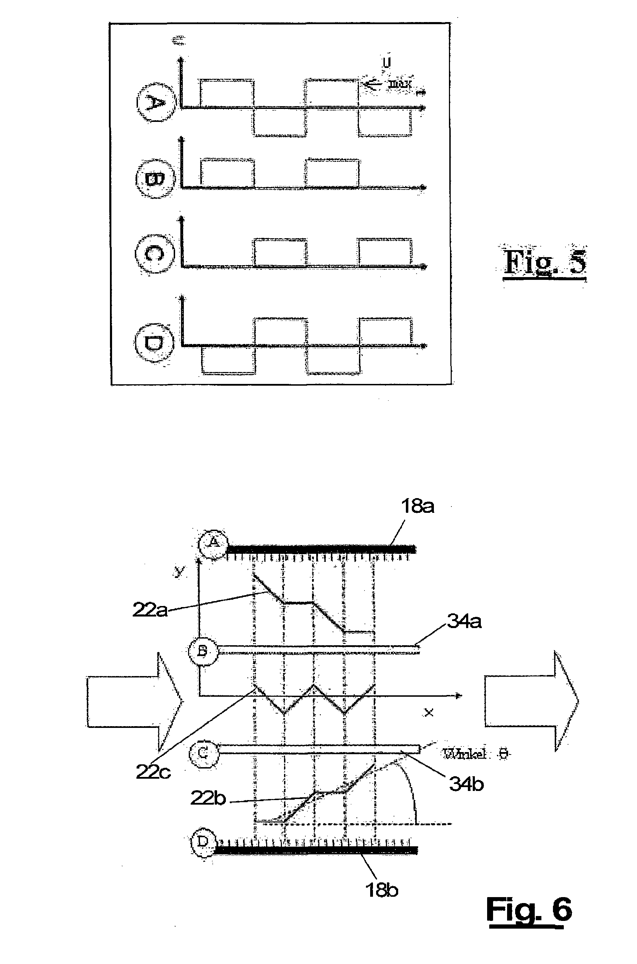

Die Ionisierungselektroden **18b** und die Elektroden-Längsgitter **34a, 34b** sind an einen nicht dargestellten Generator angeschlossen, der eine Rechteckschwingung mit hoher Spannungsamplitude im Bereich von 4000 Volt erzeugt. In den Elektroden-Längsgittern **34a, 34b** schwingt die Spannung nur im positiven Bereich, während die Schwingungen entlang der beiden außen liegenden Ionisierungselektroden **18b** sowohl im positiven als auch negativen Spannungsbereich schwingen. Dabei verlaufen die Schwingungen der linken und rechten Kanalseite (also der linken Ionisierungselektrode **18b)** sowie das benachbarten Elektroden-Längsgitter **34a** gegenüber der rechten Ionisierungselektrode **18b** und dem rechten Elektroden-Längsgitter **34b** um einen Phasenwinkel von 90° versetzt, wie in **Figur 5** dargestellt ist.

In **Figur 3** ist die Bewegungsbahn eines stark elektronenaffinen Partikels **22** sowie eines neutralen Partikels **24** dargestellt, der ohne Beeinflussung (bis auf strömungsmechanische Umströmungsbewegungen aufgrund der Existenz der Gitter oder des Querstromkanals **38)** gradlinig durch den Strömungskanal **12b** wandert.

Die stark elektronenaffinen Partikel werden bei den Ionisierungsspitzen **36** durch die Koronaentladung indirekt geladen und aufgrund der nunmehr gegebenen elektrisch negativen Ladung im elektrischen Feld zwischen den Ionisierungselektroden **18b** und den Elektrodenlängsgittern **34** beschleunigt. Die elektrostatische Kraft wechselt wegen der Rechteckschwingung der Spannung mit der Spannungsfrequenz die Richtung. Durch diese Anordnung wirkt das elektrische Feld in den Außenbereichen (also zwischen den Ionisierungselektroden) und den benachbarten Elektroden-Längsgittern **34** zentrierend analog zu einer monotonen Funktion in der Mathematik. Im Innenbereich zwischen den beiden Elektroden-Längsgittern **34a, 34b** wechselt die Kraft mit dem gleichen Betrag was theoretisch in einer Flugbahn in Form einer Dreieckschwingung resultiert, welche effektiv neutral bleibt. Diese Anordnung hat den Effekt, dass alle elektrisch negativ geladenen Partikel in dem zentralen Bereich fokussiert werden und am Ende der Ionisierungsstrecke durch die Öffnung **40** in den Querstromkanal **38** eintreten und dort auf die Kollektorelektrode **20b** treffen, wo diese neutralisiert werden und dann aufgrund des Gasstroms im Querstromkanal mitgerissen werden.

Die elektrisch isolierende Schicht auf der Kollektorelektrode **20b** besteht bei diesem Ausführungsbeispiel aus einem 4x4 cm² Silizium-Wafer, der nach einer gewissen Betriebszeit entnommen und der thermischen Desorption zugeführt wird.

**Figur 6** zeigt eine schematische Darstellung, bei der die Bewegungsbahn von stark elektronenaffinen Partikeln abhängig von dem Eintrittspunkt relativ zu den Gittern **18, 34** beim Durchtritt durch die Gitter dargestellt sind. Ein stark elektronenaffiner Partikel **22a,** der zwischen der Ionisierungselektrode **18a** und dem benachbarten Elektroden-Längsgittern **34a** eintritt, wird aufgrund des in Fig. 5 gezeigten Spannungsverlaufes während der Phasen, in denen an der Ionisierungselektrode **18a** eine negative Spannung anliegt, nach innen zur Elektroden-Längsgittern **34a** bewegt und während der alternativen Phase, bei der an beiden Elektroden **18a, 34a** positive Spannungen anliegen, im wesentlichen unbeeinflusst in Bewegungsrichtung des Luftstromes mitgerissen so dass sich über viele Spannungsperioden eine resultierende Bewegung zur Mitte hin ergibt. Auf der anderen Seite, also zwischen Ionisierungselektrode **18b** und dem benachbarten Elektroden-Längsgittern **34b,** gilt für einen stark elektronenaffinen Partikel **22b** spiegelbildlich das gleiche, auch dieser Partikel **22b** wird zur Mitte hin bewegt. Im Mittel ergibt sich dabei eine Strömungsrichtung, die in einem Winkel Θ zur Strömungsrichtung verläuft.

Für einen stark elektronenaffinen Partikel **22c**, der zwischen den beiden Elektroden-Längsgittern **34a, 34b** eintritt, wird aufgrund der alternativ an diesen Gittern anliegenden positiven Spannung im Rhythmus der Spannungsperiode im wesentlich zickzackartig bewegt, im Mittel ist seine Bewegungsbahn also parallel zur Strömungsrichtung.

Daher werden insgesamt gesehen die stark elektronenaffinen Partikel **22** zur Mitte hin bewegt, so dass diese in dem in Fig. 3 gezeigten Querstromkanal **38** aufgefangen werden können.

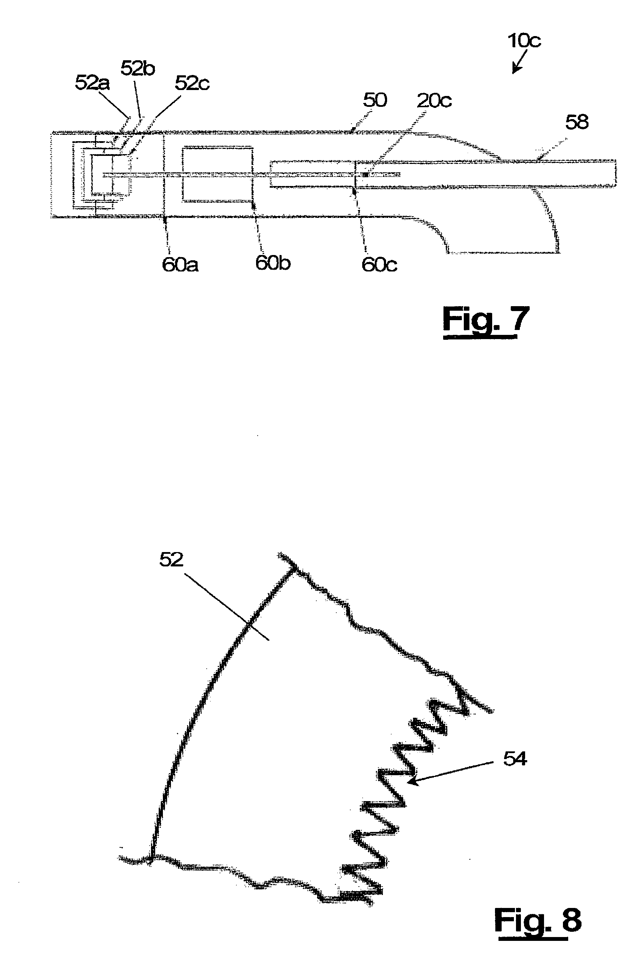

**Fig. 7** zeigt eine schematische Schnittdarstellung einer dritten Ausführungsform einer Partikelsammelvorrichtung **10c,** die im wesentlichen aus einem runden Mantelrohr **50** besteht, das im Bereich des einlassseitigen Endes drei konzentrische und in Strömungsrichtung von außen nach innen axial versetzte Ionisationsspitzenringe **52a, 52b, 52c** aufweist. Das strömungsaustrittseitige Ende des Mantelrohrs **50** ist in der gezeigten Ausführungsform gekrümmt. Die Ionisationsspitzenringe **52a, 52b, 52c** haben eine gezackte Hinterkante **54** mit scharfen Spitzen, um Ionisationsspitzen auszubilden, wie schematisch in **Fig. 8** dargestellt ist. An den Ionisationsspitzenringen **52a, 52b, 52c** liegen jeweils negative Spannungen an, die vom ersten zum dritten Ionisationsspitzenring **52a, 52c** hin abnehmen.

Koaxial zum Mantelrohr **50** ist eine stabförmige, passivierte Kollektorelektrode **20c** vorgesehen, die in ein zum Mantelrohr **50** konzentrisches Sammelrohr **58** mündet. Ferner sind drei axial hintereinander angeordnete zum Mantelrohr **50** konzentrische Feldringe **60a, 60b, 60c** mit abnehmenden Durchmessern vorgesehen, die jeweils negative Spannungen aufweisen, die vom ersten zum dritten Feldringe **60a 60c** hin abnehmen.

Im Betrieb tritt der Luftstrom in Fig 8 am linken Ende in das Mantelrohr **50** ein. Durch die an den Ionisationsspitzenringen **52a, 52b, 52c** herrschenden Bedingungen werden die stark elektronenaffinen Partikel dort indirekt negativ aufgeladen und anschließend durch die Abstoßungskraft im Inneren der negativ geladenen Feldringe **60a, 60b, 60c** zur Mittelachse bzw. der zentrischen Kollektorelektrode **20c** hin bewegt. Also zu dem durch die Strömung vorgegebenen Bewegungsvektor addieren sich die durch die elektrischen Felder auf die stark elektronenaffinen Partikel bewirkten Bewegungsvektoren, so dass diese sich auf einer gekrümmten Bahn zur Kollektorelektrode bewegen. Durch die Passivierung der Kollektoroberfläche können die Partikel ihre negative Ladung nicht abgeben und bleiben in Folge der Passivierung dort haften. Mit der Zeit sammeln sich dort dann entsprechend viele Partikel an.

Um die Partikel vom Kollektor zu entfernen bestehen zwei bevorzugte Möglichkeiten. In einer Ausführungsform wird die Kollektorelektrode **20c** kurzzeitig negativ geladen, so dass die Partikel sich von der Kollektorelektrode ein Stück entfernen und durch die weitgehend laminare Strömung im Mantelrohr **50** bzw. dem Sammelrohr **58** mitgenommen werden. Dabei muss der Zeitraum der negativen Aufladung der Kollektorelektrode **20c** so kurz sein, dass sich die geladenen Partikel nur wenig von der Kollektroelektrode **20c** entfernen.

Alternativ kann die Kollektorelektrode **20c** axial entfernbar ausgebildet sein und - in der Zeichnung nach rechts - entnommen werden wobei dann eine Abstreifeinrichtung nötig ist, welche die Partikel von der Kollektorelektrode **20c** abstreift. Bei dieser Ausführung kann auf das Sammelrohr **58** verzichtet werden.

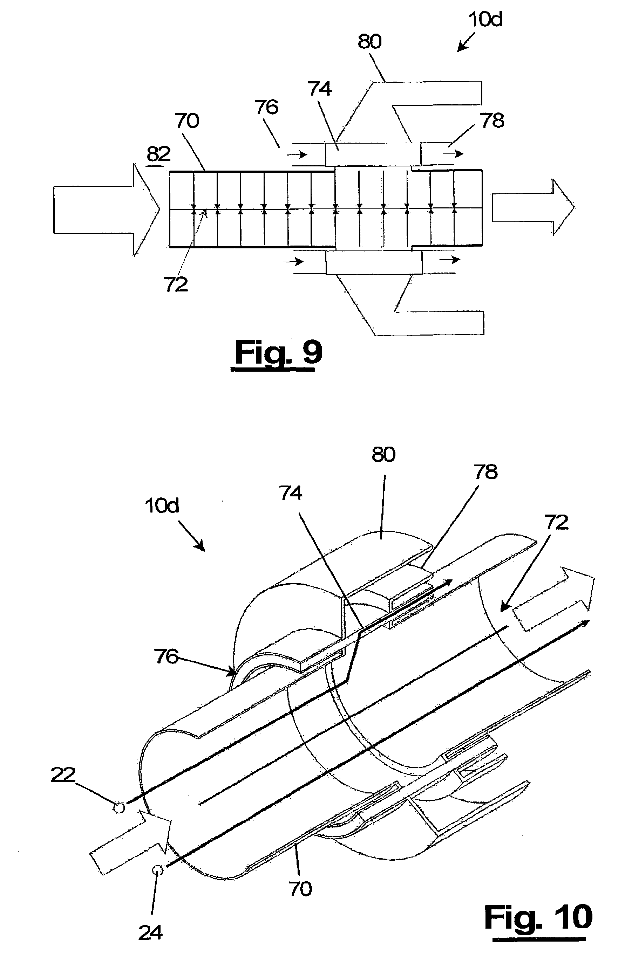

**Fig. 9 und 10** zeigen eine schematische Schnittdarstellung bzw. perspektivische Darstellung einer vierten bevorzugten Ausführungsform einer Partikelsammelvorrichtung **10d,** die im wesentlichen aus einer positiv geladenen Mantelelektrode **70** besteht, in dem mittig eine negativ geladene Koronaelektrode **72** angeordnet ist. Im Abstand zum eintrittsseitigen Ende weißt die Mantelelektrode **70** einen ringförmigen Auslass auf, der von einem Vliesfilter **74** umgeben ist. Dieses Ringelement **74** weist einen Querstromeinlass **76** sowie einen Querstromauslass **78** auf, sowie einen Saugkanal **80.** In Fig. 10 ist der Weg eines stark elektronenaffinen Partikels **22** sowie eines gering elektronenaffinen Partikels **24** dargestellt. Der partikelbeladene Strom tritt bei **82** in die Mantelelektrode **70** ein, wobei die stark elektronenaffinen Partikel an der Koronaelektrode **72** indirekt aufgeladen und anschließend nach radial außen bewegt werden. Über den Saugkanal **80** wird Luft aus dem Hauptstrom abgesaugt, wobei die in Wandungsnähe konzentrierten elektrisch geladenen Partikel mitgenommen und in den Vliesfilter **74** eintreten und dort hängen bleiben. Der Vliesfilter **74** hat dabei von innen nach außen eine abnehmende Porengröße so dass die größeren Partikel weiter innen und die kleineren weiter außen hängen bleiben.

Nach einer ausreichenden Zeitspanne wird vom Querstromeinlass **76** zum Querstromauslass **78** ein Gasstrom quer zur bisherigen Durchströmungsrichtung durch das Vliesfilter **74** geleitet, wodurch die im Vliesfilter **74** befindlichen stark elektronenaffinen Partikel daraus entfernt und über den Querstromauslass **78** einer nicht dargestellten Messungeinrichtung zugeführt werden.

## Patentansprüche

1. Vorrichtung zur Sammlung von stark elektronenaffinen Partikeln, insbesondere von Sprengstoffpartikeln, aus einem Gas, mit einem Strömungskanal (12, 50, 70), in dem mindestens eine elektrisch positive Kollektorelektrode (20a, 20b, 20c) und mindestens eine Ionisierungselektrode (18a, 18b, 52, 72) angeordnet sind, zwischen denen ein elektrisches Feld herrscht, so dass die stark elektronenaffinen Partikel (22) durch Koronaentladung an der Ionisierungselektrode (18a, 18b, 52, 72) indirekt aufladbar und zur Kollektorelektrode (20a, 20b, 20c) bewegbar sind, **dadurch gekennzeichnet, dass** vor der Kollektorelektrode (20a, 20b, 20c) eine elektrisch isolierende Schicht angeordnet ist, auf der sich die Partikel anlagern.

2. Partikelsammelvorrichtung nach Anspruch 1, **dadurch gekennzeichnet, dass** die elektrisch isolierende Schicht entfernbar ist.

3. Partikelsammelvorrichtung nach Anspruch 1, **dadurch gekennzeichnet, dass** die Kollektorelektrode (20b) in einem gasdurchströmten Querstromkanal (38) angeordnet ist, und mittels des durchströmenden Gases die an der Kollektorelektrode (20b) neutralisierten Partikel mitnehmbar sind.

4. Partikelsammelvorrichtung nach Anspruch 1, **dadurch gekennzeichnet, dass** eine Verdampfungseinrichtung vorgesehen ist, welche auf der Kollektorelektrode (20a, 20b, 20c) angesammelte Partikel verdampft.

5. Partikelsammelvorrichtung nach Anspruch 1, **dadurch gekennzeichnet, dass** die mindestens eine Kollektorelektrode (20a) am einen Ende des Strömungskanals (12) angeordnet ist und beabstandet davon mindestens ein Gaseinlaufkanal (14) in den Strömungskanal (12) mündet, ferner der Kollektorelektrode (20a) bezüglich des Gaseinlaufkanals (14) gegenüberliegend die mindestens eine Ionisierungselektrode (18) im Strömungskanal (12) angeordnet ist.

6. Partikelsammelvorrichtung nach Anspruch 4, **dadurch gekennzeichnet, dass** zwei sich bezüglich der Mittelachse des Strömungskanals (12) gegenüberliegende Gaseinlaufkanäle (14a, 14b) vorgesehen sind.

7. Partikelsammelvorrichtung nach Anspruch 4, **dadurch gekennzeichnet, dass** die Ionisierungselektrode ein Elektrodengitter (18a) umfasst, das im Wesentlichen quer im Strömungskanal (12) angeordnet ist.

8. Partikelsammelvorrichtung nach einem der vorherigen Ansprüche, **dadurch gekennzeichnet, dass** stromab des Gaseinlasses eine Wasserdampf-Einspritzvorrichtung vorgesehen ist, durch welche sich Wasserdampf an den Partikeln anlagert.

9. Partikelsammelvorrichtung nach einem der vorherigen Ansprüche, **dadurch gekennzeichnet, dass** hinter der Kollektorelektrode (20) eine Strahlungseinrichung angeordnet ist, um diese aufzuheizen und die gesammelten Partikel in Dampfform zu überführen.

10. Partikelsammelvorrichtung nach einem der vorherigen Ansprüche, **dadurch gekennzeichnet, dass** die Kollektorelektrode (20) in einem zur Partikelströmung hin offenen Kollektorraum angeordnet ist, so dass diese nicht vom Gasstrom umströmt ist.

11. Partikelsammelvorrichtung nach Anspruch 10, **dadurch gekennzeichnet, dass** der Kollektorraum in einem gasdurchströmten Querstromkanal angeordnet ist und die Partikel im Kollektorraum einem Detektor zuführt.

12. Partikelsammelvorrichtung nach einem der vorherigen Ansprüche, **dadurch gekennzeichnet, dass** diese mit einer Verdampfungseinrichtung verbunden ist, in der die gesammelten Partikel verdampfbar sind.

## Claims

1. A device for collecting particles with a strong electron affinity, in particular explosive particles, from a gas, with a flow channel (12, 50, 70), which incorporates at least one electrically positive collector electrode (20a, 20b, 20c) and at least one ionizing electrode (18a, 18b, 52, 72), between which an electrical field prevails, so that the particles with a strong electron affinity (22) can be indirectly charged via corona discharge at the ionizing electrode (18a, 18b, 52, 72) and moved to the collector electrode (20a, 20b, 20c), **characterized in that** an electrically insulating layer upon which the particles become deposited is arranged in front of the collector electrode (20a, 20b, 20c).

2. The particle collecting device according to claim 1, **characterized in that** the electrically insulating layer can be removed.

3. The particle collecting device according to claim 1, **characterized in that** the collector electrode (20b) is arranged in a cross channel (38) carrying a flow of gas, and that particles neutralized on the collector electrode (20b) can be entrained by the gas flowing through.

4. The particle collecting device according to claim 1, **characterized in that** an evaporator is provided that evaporates particles that have accumulated on the collector electrode (20a, 20b, 20c).

5. The particle collecting device according to claim 1, **characterized in that** the at least one collector electrode (20a) is arranged at one end of the flow channel (12), and at least one gas inlet channel (14) spaced apart from the latter empties into the flow channel (12), with the at least one ionizing electrode (18) opposite the collector electrode (20a) relative to the gas inlet channel (14) being situated in the flow channel (12).

6. The particle collecting device according to claim 4, **characterized in that** two gas inlet channels (14a, 14b) lying opposite each other relative to the central axis of the flow channel (12) are provided.

7. The particle collecting device according to claim 4, **characterized in that** the ionizing electrode encompasses an electrode gate (18a), which is essentially transversely arranged in the flow channel (12).

8. The particle collecting device according to one of the preceding claims, **characterized in that** a water vapour injector is provided downstream from the gas inlet, and causes water vapour to become deposited on the particles.

9. The particle collecting device according to one of the preceding claims, **characterized in that** a radiator is arranged behind the collector electrode (20) so as to heat the latter and transform the accumulated particles into vapour form.

10. The particle collecting device according to one of the preceding claims, **characterized in that** the collector electrode (20) is arranged in a collector space open toward the particle flow, so that the gas does not flow around it.

11. The particle collecting device according to claim 10, **characterized in that** the collector space is arranged in a cross channel that carries a flow of gas, and guides the particles in the collector space to a detector.

12. The particle collecting device according to one of the preceding claims, **characterized in that** it is connected with an evaporator in which the accumulated particles can be evaporated.

## Revendications

1. Dispositif de collecte de particules à affinité électromagnétique, notamment de particules d'explosif, dans un gaz, comportant un canal d'écoulement (12, 50, 70), dans lequel au moins une électrode collectrice électriquement positive (20a, 20b, 20c) et au moins une électrode d'ionisation (18a, 18b, 52, 72) sont disposées, entre lesquelles un champ électrique prédomine, de telle sorte que les particules à forte affinité électromagnétique (22) puissent être chargées indirectement par effet corona sur l'électrode d'ionisation (18a, 18b, 52, 72) et puissent être déplacées vers l'électrode collectrice (20a, 20b, 20c), **caractérisé en ce que** une couche isolante électriquement est disposée avant l'électrode collectrice (20a, 20b, 20c), sur laquelle les particules s'accumulent.

2. Dispositif de collecte de particules selon la revendication 1, **caractérisé en ce que** la couche isolante électriquement peut être retirée.

3. Dispositif de collecte de particules selon la revendication 1, **caractérisé en ce que** l'électrode collectrice (20b) est disposée dans un canal d'écoulement transversal (38) traversé par un écoulement de gaz, et les particules neutralisées peuvent être emportées au moyen du gaz s'écoulant sur l'électrode collectrice (20b).

4. Dispositif de collecte de particules selon la revendication 1, **caractérisé en ce que** un dispositif d'évaporation est prévu, lequel évapore les particules accumulées sur l'électrode collectrice (20a, 20b, 20c).

5. Dispositif de collecte de particules selon la revendication 1, **caractérisé en ce que** au moins une électrode collectrice (20a) est disposée à une extrémité du canal d'écoulement (12) et au moins un canal d'introduction de gaz (14) espacé de celle-ci débouche dans le canal d'écoulement (12), en outre l'électrode collectrice (20a) est disposée en vis-à-vis d'au moins une électrode d'ionisation (18) par rapport au canal d'écoulement de gaz (14) dans le canal d'écoulement (12).

6. Dispositif de collecte de particules selon la revendication 4, **caractérisé en ce que** deux canaux d'introduction de gaz (14a, 14b) en vis-à-vis de part et d'autre de l'axe médian du canal d'écoulement (12) sont prévus.

7. Dispositif de collecte de particules selon la revendication 4, **caractérisé en ce que** l'électrode d'ionisation comprend une grille d'électrode (18a), qui est disposée essentiellement transversalement dans le canal d'écoulement (12).

8. Dispositif de collecte de particules selon une des revendications précédentes, **caractérisé en ce que** en aval de l'admission de gaz, un dispositif de pulvérisation de vapeur d'eau est prévu, par l' intermédiaire duquel la vapeur d'eau se dépose sur les particules.

9. Dispositif de collecte de particules selon une des revendications précédentes, **caractérisé en ce que** derrière l'électrode collectrice (20) un dispositif d'irradiation est disposé, afin de chauffer celle-ci et de transformer les particules collectées en vapeur.

10. Dispositif de collecte de particules selon une des revendications précédentes, **caractérisé en ce que** l'électrode collectrice (20) est disposée dans un espace collecteur ouvert dans la direction du flux de particules, de telle sorte que celle-ci ne soit pas baignée par le flux de gaz.

11. Dispositif de collecte de particules selon la revendication 10, **caractérisé en ce que** l'espace collecteur est disposé dans un canal d'écoulement transversal traversé par un écoulement de gaz et les particules dans l'espace collecteur sont introduites dans un détecteur.

12. Dispositif de collecte de particules selon une des revendications précédentes, **caractérisé en ce que** celui-ci est relié à un dispositif d'évaporation, dans lequel les particules collectées peuvent être évaporées.
